# EUROPEAN PATENT SPECIFICATION

(11) **EP 1 871 243 B1**
(45) Date of publication and mention of the grant of the patent: **15.07.2009**
(21) Application number: 06736814.2
(22) Date of filing: 03.03.2006
(51) Int. Cl.: A61B 17/22, A61F 2/01, A61M 25/10, A61M 25/00, A61B 18/26

(54) **EXPANDABLE MEDICAL RETRIEVAL DEVICE**
EXPANDIERBARE MEDIZINISCHE RÜCKHOLVORRICHTUNG
DISPOSITIF D'EXTRACTION MEDICALE EXPANSIBLE

(30) Priority: 05.04.2005 US 98518
(43) Date of publication of application: 02.01.2008
(73) Proprietor: Boston Scientific Limited, Barbados, West Indies (BB)
(72) Inventor: CHENG, Eric, Bloomington, Indiana 47404 (US)
(74) Representative: Intes, Didier Gérard André
(86) International application number: PCT/US2006/007557
(87) International publication number: WO 2006/107473

(56) References cited:
- WO-A-03/024299
- US-A- 5 935 139
- US-A1- 2001 025 155

## Description

### DESCRIPTION OF THE INVENTION

This international application claims the priority of earlier filed United States Patent Application No. 11/098,518, filed April 5, 2005.

### Field of the Invention

This invention relates to medical devices and methods for retrieving objects within anatomical lumens of the body. More particularly, the invention relates to methods, and devices, for retrieving and preventing undesired migration of stones, such as urinary tract stones, gall stones, and other objects within anatomical lumens of the body, during a medical procedure.

### Background of the Invention

Medical immobilization and retrieval devices may include devices for stabilizing and/or removing organic material (e.g., blood clots, tissue, and biological concretions such as urinary, biliary, and pancreatic stones) and inorganic material (e.g., components of a medical device or other foreign matter), which may obstruct or otherwise be present within a body's anatomical lumens. For example, concretions can develop in certain parts of the body, such as in the kidneys, pancreas, and gallbladder. Minimally invasive medical procedures generally involve causing limited trauma to the tissues of a patient, and can be used to dispose of problematic concretions. Lithotripsy and ureteroscopy, for example, are used to treat urinary calculi (e.g., kidney stones) in the ureter of patients.

Lithotripsy is a medical procedure that uses energy in various forms such as acoustic shock waves, pneumatic pulsation, electrical hydraulic shock waves, or laser beams to break up biological concretions such as urinary calculi (e.g., kidney stones). The force of the energy, when applied either extracorporeally or intracorporeally, usually in focused and continuous or successive bursts, divides a kidney stone into smaller fragments that may be extracted from the body or allowed to pass through urination.

When stones are fragmented within a body tract by a lithotriptor, the stone must first be stabilized. Typically, a medical retrieval device, such as a surgical grasper or a metal wire basket, is used to capture a stone in the retrieval assembly. With the stone held in position within the retrieval assembly, a lithotriptor, such as a laser lithotriptor, comes into proximity with the stone and the stone is fragmented by the lithotriptor. After the stone is fragmented, the stone fragments can be removed by the same or a different medical retrieval device, or the fragments can be left in the body to be eliminated naturally. With the help of imaging tools such as transureteroscopic video technology and fluoroscopic imaging, the operator of the lithotripter device can monitor the progress of the medical procedure and terminate treatment when residual fragments are small enough to be voided or removed.

Intracorporeal fragmentation of urinary calculi can prove problematic in that stones and/or stone fragments in the ureter may become repositioned closer to and possibly migrate back toward the kidney, thereby requiring further medical intervention to prevent the aggravation of the patient's condition. Existing practices to control migration of stones during lithotripsy include reducing the energy or frequency of the lithotripsy, or reducing the amount or frequency of irrigation used during the procedure. Another known practice includes pushing the stone into the renal pelvis and undertaking another future procedure for its removal.

Various devices may be deployed to control migration and aid in retrieval of fragmented stones. For example, combined immobilization and retrieval devices may be deployed within a patient's body, independently, or through the working channel of an endoscope. Once deployed past the stone, the immobilization device can act as a backstop to prevent upward migration of fragments resulting from a lithotripsy procedure.

Laser lithotriptors, for example, are effective in fragmenting stones that are captured in a retrieval assembly of a medical retrieval device. One drawback of the combined use of a laser lithotriptor and a backstop and/or retrieval assembly is the susceptibility of the assembly, or parts of the assembly, to laser energy-induced damage. Damage may be caused by misfiring, misdirection, or unavoidable misalignment of the laser lithotriptor with the stone. Laser energy-induced retrieval assembly damage may cause components of the backstop and/or retrieval assembly, such as portions of a traditional metal basket, to become roughened or broken. Broken or roughened portions of the device expose sharp ends or surfaces that can traumatize the delicate internal lining of the ureter.

Known medical devices for preventing the migration of stones and fragments are often deployed beyond a stone in a configuration that partially occludes the lumen or acts as a barrier to prevent the passage of unwanted material beyond a treatment site. The occluding elements are often made of materials formed at least partially of shape-memory materials, such as, stainless steel, nitinol, copper, cobalt, vanadium, chromium, iron, or the like. The continued deployment, repositioning, and movement of these metallic materials within a patient's body lumen can often cause undesired irritation and unnecessary trauma to the patient's body tract.

Thus, it is desirable to have alternative methods and devices for preventing upward migration of fragments, and extracting such fragments while limiting trauma to the patient. Other medical devices of the prior art are disclosed in documents WO 03/024299 and US 2001/025155.

### SUMMARY OF THE INVENTION

Embodiments of the present invention are directed to medical devices for immobilization and/or retrieval of objects within anatomical lumens of the body that obviate one or more of the limitations and disadvantages of prior immobilization and retrieval devices. A medical device according to the invention is defined in claim 1.

In one embodiment, the medical device includes a sheath having a lumen, a distal end, and a proximal end. An elongate member is provided including an expansible member connected to a distal portion of the elongate member. The elongate member and expansible member are movable relative to the sheath to achieve a first state of the expansible member when the expansible member is within the lumen of the sheath and an expanded state when the expansible member extends from the distal end of the sheath. The expansible member comprises a material that expands to the expanded state due to the presence of fluid.

In various embodiments, the medical device may include one or more of the following additional features: a medical device wherein the first state is a compressed state; wherein the elongate member is a flexible wire; a handle at a proximal end of the medical device configured to control axial movement of the expansible member relative to the sheath; wherein the expansible member has a proximal end and a distal end, and markers are positioned along the elongate member proximate the distal and proximal ends of the expansible member; wherein the expansible member has a tapered proximal end to facilitate movement of the expansible member into the lumen of the sheath; wherein the expansible member has a tapered distal end to facilitate movement of the expansible member out of the lumen of the sheath; wherein the expansible member comprises a material that exhibits an expansion/compression size ratio of approximately 10:1; wherein the expansible member comprises Poly-vinyl Alcohol (PVA); wherein the expansible member comprises a sponge; wherein the expansible member defines holes formed therein for passing irrigation therethrough in the expanded state; wherein the expansible member comprises a material less susceptible to laser-energy induced damage than alloys of nickel/titanium, copper, cobalt, vanadium, chromium, and iron; wherein the sheath includes a longitudinal split along the length such that the sheath can be separated from the expansible member and elongate member by separation along the split; an second sheath for tracking over a proximal end of the elongate member after removal of the first sheath; wherein the second sheath has a lumen larger than the lumen of the first sheath and distal end for receiving the expansible member; wherein the additional sheath includes a tapered distal end extending to an enlarged distal opening.

Another embodiment of the invention is directed to a medical device including a sheath having a lumen, a distal end, and a proximal end. An elongate member is provided including an expansible member connected to a distal portion of the elongate member. The elongate member and expansible member are movable relative to the sheath to achieve a first state of the expansible member when the expansible member is within the lumen of the sheath and an expanded state when the expansible member extends from the distal end of the sheath. In addition, the expansible member comprises a sponge.

Another embodiment of the invention is directed to a method for retrieving material in a body. The method includes providing a medical device including a sheath having a lumen, a distal end, and a proximal end. An elongate member is provided including an expansible member connected to a distal portion of the elongate member. The elongate member and expansible member are movable relative to the sheath to achieve a first state of the expansible member when the expansible member is within the lumen of the sheath and an expanded state when the expansible member extends from the distal end of the sheath. The expansible member comprises a material that expands to the expanded state due to the presence of fluid. The method further comprises inserting the medical device into an anatomical lumen of the body, with the expansible member of the medical device in a collapsed configuration; positioning the distal end of the sheath beyond the material to be immobilized; and moving the sheath relative to the elongate member and expansible member, such that the expansible member is expanded to the expanded second state outside the distal end of the sheath and at least partially occludes the anatomical lumen.

In various embodiments, the method may include one or more of the following additional features: performing a lithotripsy procedure on the material; irrigating the lumen of the body; retrieving the immobilized material by proximally pulling the elongate member through the anatomical lumen with the expansible member in the expanded state; retracting the expansible member by moving the sheath relative to the elongate member and expansible member, such that the expansible member is retracted to a compressed state inside the lumen of the sheath; wherein anatomical lumen includes an interior surface and the expansible member expands to contact the interior surface of the anatomical lumen; wherein the expansible member comprises a material that exhibits an expansion/compression size ratio of approximately 10:1; wherein the expansible member comprises Poly-vinyl Alcohol (PVA); wherein the expansible member comprises a sponge; wherein the expansible member defines holes formed therein for passing irrigation therethrough in the expanded state; wherein the expansible member comprises a material less susceptible to laser-energy induced damage than alloys of nickel/titanium, copper, cobalt, vanadium, chromium, and iron; wherein the expansible member has a proximal end and a distal end, and markers are positioned along the elongate member proximate the distal and proximal ends of the expansible member; wherein the steps of positioning and moving further include visualizing the position of the markers through a medical imaging device; removing the sheath by completely backing off the sheath from the elongate member; providing a second sheath for tracking over a proximal end of the elongate member after removal of the first sheath, the second sheath having a lumen larger than the lumen of the first sheath and a distal end for receiving the expansible member; wherein the second sheath includes a tapered distal end extending to an enlarged distal opening; tracking the second sheath over the proximal end of the elongate member; retrieving the immobilized material within the lumen of the second sheath by positioning the material between the distal end of the second sheath and the expansible member and then moving the second sheath relative to the elongate member, such that the expansible member is retracted to a compressed state inside the lumen of the second sheath along with the material.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this specification, illustrate several embodiments of the invention and together with the description, serve to explain the principles of the invention.

FIG. 1A is a partial side cross-sectional view of a medical immobilization/retrieval device in an undeployed position according to an embodiment of the invention.

FIG. 1B is a partial side cross-sectional view of the medical immobilization/retrieval device of FIG. 1A in a partially deployed position.

FIG. 2A illustrates the insertion of a distal portion of the medical immobilization/retrieval device into a ureter containing a kidney stone, according to an embodiment of the invention.

FIG. 2B illustrates the medical immobilization/retrieval device of FIG. 2A having an expansible member deployed beyond a kidney stone in the ureter, according to an embodiment of the invention.

FIG. 2C illustrates the proximal movement of a deployed expansible member of a medical immobilization/retrieval device within the ureter, according to an embodiment of the invention.

FIG. 2D illustrates a medical immobilization/retrieval device having an expansible member deployed beyond a kidney stone in the ureter and a lithotripsy device positioned proximal to the kidney stone, according to an embodiment of the invention.

FIG. 2E is similar to FIG. 2D except that FIG. 2E shows the kidney stone after being fragmented by a medical lithotripsy device, according to an embodiment of the invention.

FIG. 2F illustrates the partial retraction of an expansible member of the medical immobilization/retrieval device of FIGS. 2A-2E, according to an embodiment of the invention.

FIG. 3A illustrates an alternative medical immobilization/retrieval device, according to an embodiment of the invention.

FIG. 3B illustrates a retrieval procedure for the medical immobilization/retrieval device of FIG. 3A, according to an embodiment of the invention.

### DESCRIPTION OF THE EMBODIMENTS

Reference will now be made in detail to the present exemplary embodiments of the invention illustrated in the accompanying drawings. Wherever possible, the same reference numbers will be used throughout the drawings to refer to the same or like parts.

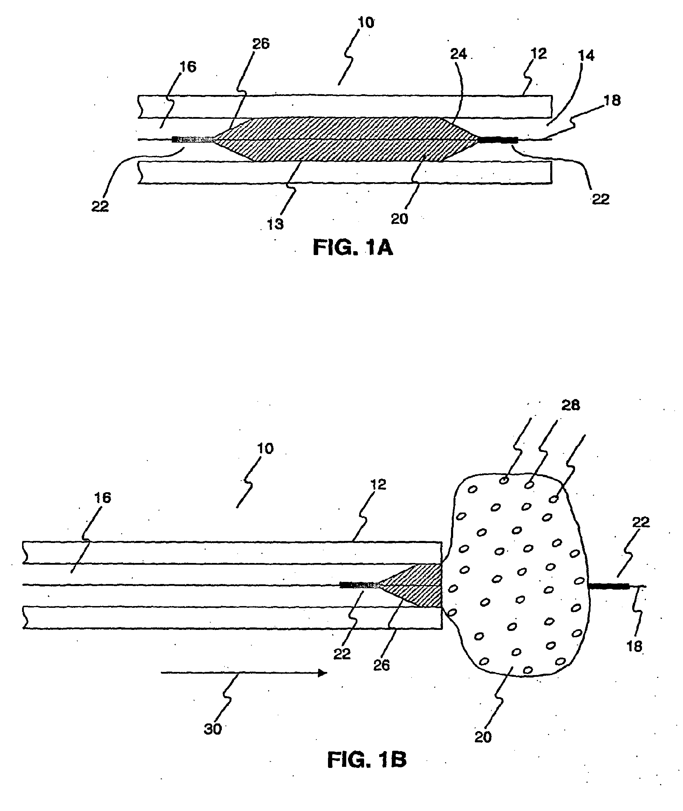

FIG. 1A illustrates a medical device 10 having a sheath 12 and an inner immobilization device 13, according to an embodiment of the invention. Sheath 12 includes a distal opening 14 and an internal sheath lumen 16. The sheath 12 can be manufactured out of materials such as polytetrafluoroethylene (PTFE), polyimide, nylon, or other polymers with column strength and flexibility. In addition, the sheath 12 can be braided to add additional kink resistance.

The immobilization device 13 is initially housed within the sheath lumen 16. The immobilization device 13 includes an elongate member 18 and an expansible member 20 attached along a length of the elongate member 18. The elongate member 18 may be a wire, cable, or other suitable flexible elongate structure movable within the lumen 16 of the sheath 12. Elongate member 18 may include coils attached to improve flexibility and aid in positioning the device within tortuous passages of a patient's body. Marker bands 22, such as radiopaque markers or other suitable markers, are attached to the elongate member 18 at the distal and proximal ends of the expansible member 20. The expansible member 20 is constrained in a collapsed state by sheath 12 and is movable relative to the sheath 12 within lumen 16. The expansible member 20 may include a tapered distal portion 24 and a tapered proximal portion 26 to facilitate the movement of the expansible member 20 in forward deployment from within the sheath 12 and in retraction back within the lumen 16.

As seen in FIG. 1B, the immobilization device 13 is configured for forward movement relative to the sheath 12, in the direction of arrow 30. The sheath 12 and expansible member 20 are movable relative to each other in order to achieve a first, compressed, collapsed state (FIG. 1A) in which the expansible member 20 is compressed within the internal lumen 16 of the sheath 12 and a second state (FIG. 1 B) in which the expansible member extends from the distal opening 14 of the sheath 12 and expands to greater size.

The expansible member 20 may consist of a material that expands in the presence of fluid when unconstrained by the sheath 12. For example, expansible member 20 can be formed of a Poly-vinyl Alcohol (PVA) sponge and may have an expansion ratio of approximately 10:1. A Poly-vinyl Alcohol having other suitable expansion ratios may be used. In addition to Poly-vinyl Alcohol, the expansible member 20 can be formed of any material suitable for placement within an anatomical lumen of a patient's body that exhibits a high expansion/compression ratio. Suitable alternative materials may include, but are not limited to, Soft Polyurethane or Polyethylene Foam. The use of a Poly-vinyl Alcohol material, or the like, is advantageous in that such material is less susceptible to laser-energy induced damage than other immobilization/retrieval device materials, such as traditional metals and alloys thereof.

Figure 1B illustrates a distal portion of expansible member 20 being deployed beyond the distal opening 14 of sheath 12. In the presence of liquid, such as, for example, upon deployment within the ureter of a patient, the unconstrained portion of the expansible member 20 will absorb fluid and expand according to the physical properties of the expansible member. As noted above, the immobilization device 13 is configured for movement relative to the sheath 12. Any such relative movement may be controlled by a user at a proximal end of the medical device 10. In one configuration, the elongate member 18 and sheath 12 may be coupled to any suitable proximal handle, including those known to one skilled in the art. The proximal end of the elongate member 18 may be connected to a movable internal portion of a handle at the proximal end of device 10, such that movement of the movable internal portion will move the immobilization device 13 relative to sheath 12 between expanded and collapsed states. Alternatively, in another configuration, the proximal end of the sheath 12 may be connected to a movable internal portion of a handle at the proximal end of device 10, such that movement of the movable internal portion will extend the sheath 12 over the immobilization device 13 and thereby collapse the expansible member 20.

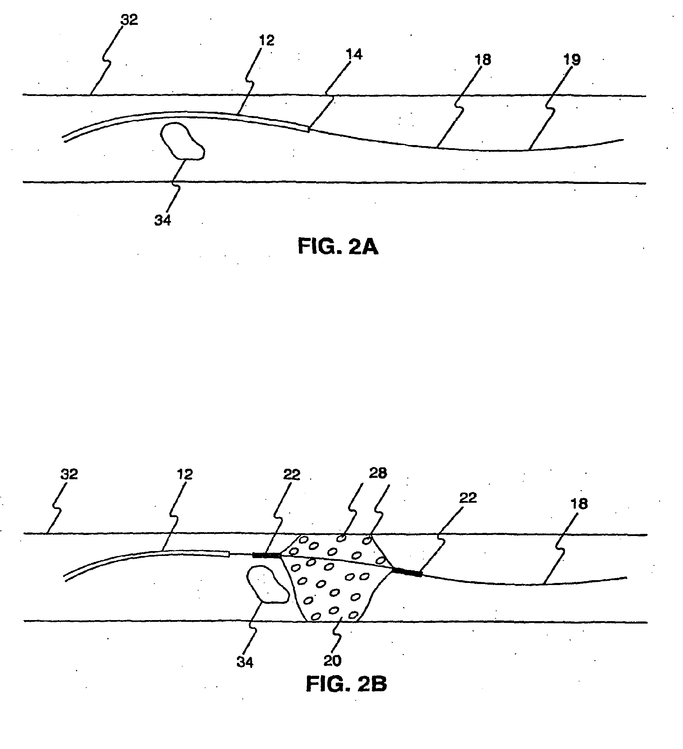

FIGS. 2A-2F illustrate a system and method for retrieving, immobilizing, and/or preventing migration of objects in anatomical lumens during a medical procedure. Referring to FIG. 2A, medical device 10 may be positioned at an internal treatment site, for example, such as within a patient's ureter 32. The positioning may be performed by any suitable method known in the art, and may include known imaging and viewing techniques. A distal portion 19 of elongate member 18 may extend outside of sheath 12 beyond the distal opening 14 and function as a guidewire for accurate placement of the medical device 10. Accordingly, the distal portion 19 of elongate member 18 may be more flexible than the remaining proximal section of device 13 (including sheath 12 and elongate member 18) to assist in positioning the elongate member 18, while preventing damage to a patient's internal tissues.

As seen in FIG. 2A, the distal portion 19 of elongate member 18 and the distal opening 14 of sheath 12 are positioned within ureter 32, beyond a kidney stone 34. Referring to FIG. 2B, elongate member 18 is then moved forward relative to sheath 12, thereby deploying the expansible member 20 within the ureter 32. For example, FIG. 2B shows that sheath 12 has been moved proximally to deploy member 20. No longer constrained by the sheath 12 and due to the presence of fluid, the expansible member 20 achieves its expanded second state beyond the kidney stone 34. Marker bands 22, attached to the elongate member 18, may comprise bands of radiopaque material for designating the position of the expansible member 20 prior to, during, and/or after positioning, using standard medical imaging technology. FIG. 2B further illustrates that in an expanded state, expansible member 20 fills a portion of ureter 32 while still adjusting to comply with contours and turns that may exist along the lumen within which the expansible member is deployed.

In embodiments where the expansible member consists of a PVA sponge material, the expansible member 20 is hydrated when deployed within a patient's anatomical body lumen, thereby expanding to expose pores 28. The pore size may be varied as appropriate depending on the desired rigidity of the sponge material. Holes defining apertures (not shown), separate from the pores 28, can be manufactured in the sponge. The holes can be used for the passage of irrigation and to provide a smaller profile of the sponge in the compressed state. The hole diameter would ideally be less than 4 mm to prevent a larger stone from being imbedded in the sponge or migrating past the sponge during deployment.

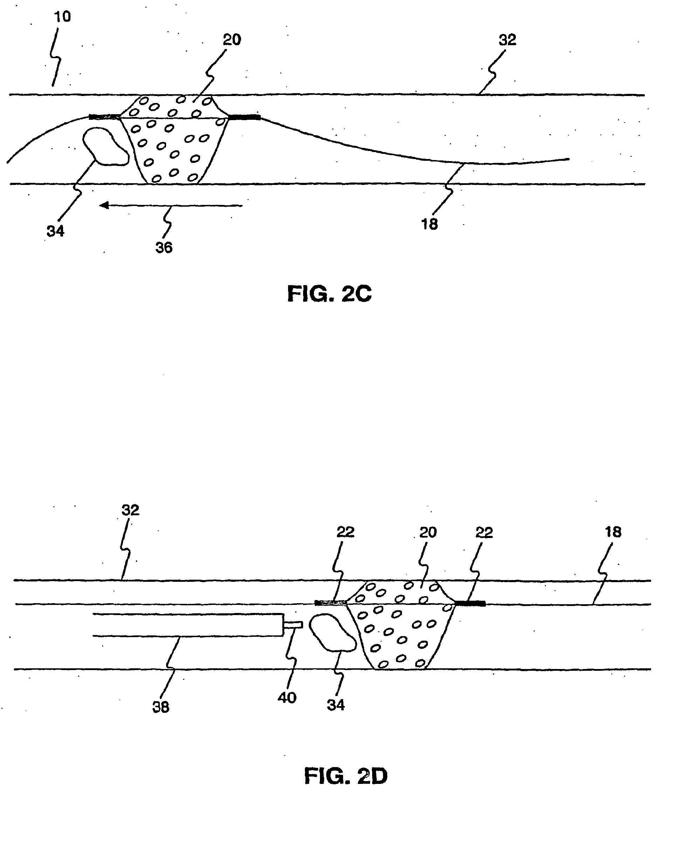

FIG. 2C illustrates the use of medical device 10 as a tool to move a stone 34 proximally along a ureter 32. In the expanded state of FIG. 2C, expansible member 20 can be moved along the ureter 32, in the direction of arrow 36, to sweep stone 34 proximally along the ureter 32. Upon the continued proximal movement of expansible member 20, stone 34 may be swept and repositioned to a new location more accessible for retrieval and removal, or swept from the ureter altogether. The material of expansible member 20, such as Poly-vinyl Alcohol, for example, is selected to be soft enough when expanded not to damage the surrounding tissue during movement and yet rigid enough to sweep or immobilize a stone 34.

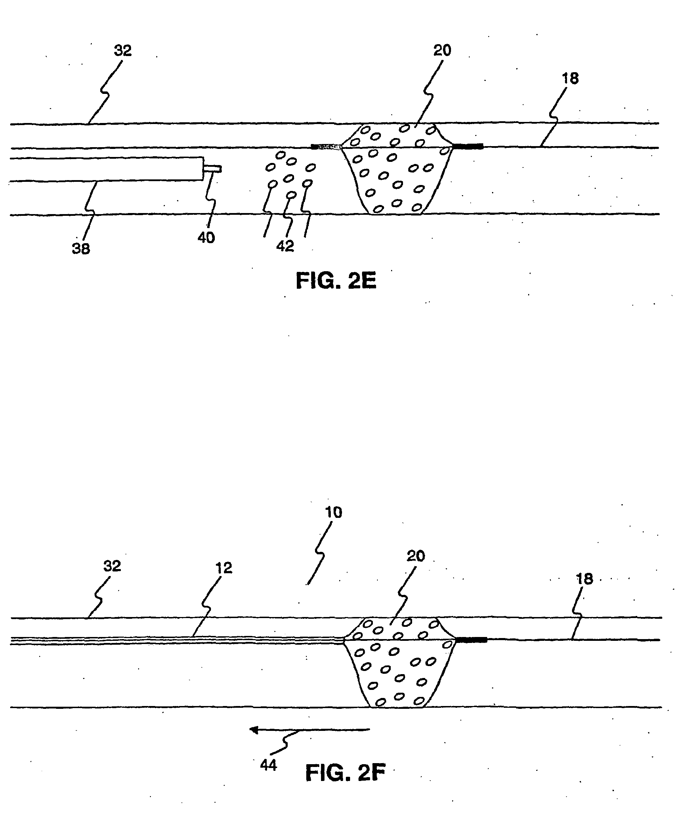

Referring to FIGS. 2D and 2E, an endoscope 38 including a lithotriptor 40 may be positioned at an internal treatment site within a patient's ureter 32 along with the deployed medical device 10. Where a concretion, such as kidney stone 34, is too large to be extracted without fragmentation, a lithotriptor 40 can be advanced through the working channel of an endoscope 38 in order to perform the fragmentation. Lithotriptor 40 may be, for example, a laser fiber for directing laser energy at kidney stone 34 in order to break down the concretion into smaller pieces to facilitate retrieval or normal passage through the bladder.

As seen in FIG. 2E, after a lithotripsy procedure is completed, kidney stone 34 is fragmented into multiple smaller stones 42. During the lithotripsy procedure, the expansible member 20 serves as a backstop to prevent migration of the smaller stones 42 beyond the immobilization device 13, thereby preventing complications resulting from the potential migration of stones 42 back into a patient's kidneys. After the fragmentation of stone 34, the lithotriptor 40 is withdrawn and the expansible member 20 can then be pulled proximally along the ureter 32 to sweep the remaining smaller stones 42 toward the bladder to be voided or repositioned to facilitate retrieval by an additional retrieval device.

If the stone fragments 42 resulting from the lithptripsy procedure are of a size capable of being accommodated within sheath 12, stones 42 may be retrieved within sheath 12 along with the immobilization device 13 upon retraction of the expansible member 20. FIG. 2F illustrates the partial retraction of expansible member 20 back within sheath 12. Upon movement of immobilization device 13 relative to the sheath 12, in the direction of arrow 44, expansible member 20 is retracted in order to achieve a collapsed configuration to facilitate removal from the patient's ureter 32. Tapered proximal portion 26, shown in FIG. 1A, of expansible member 20 facilitates the retraction of immobilization device 13 within sheath 12. Any stone fragments captured upon retraction within the expansible member 20, or between the expansible member 20 and the internal lumen 16 of the sheath 12, can be safely removed from the patient upon withdrawal of the medical device 10. In addition, stones and fragments can be immobilized and positioned for removal (in the direction of arrow 44) at the distal end of the medical device 10, between sheath 12 and expansible member 20, without necessarily being captured within internal lumen 16 of the sheath 12.

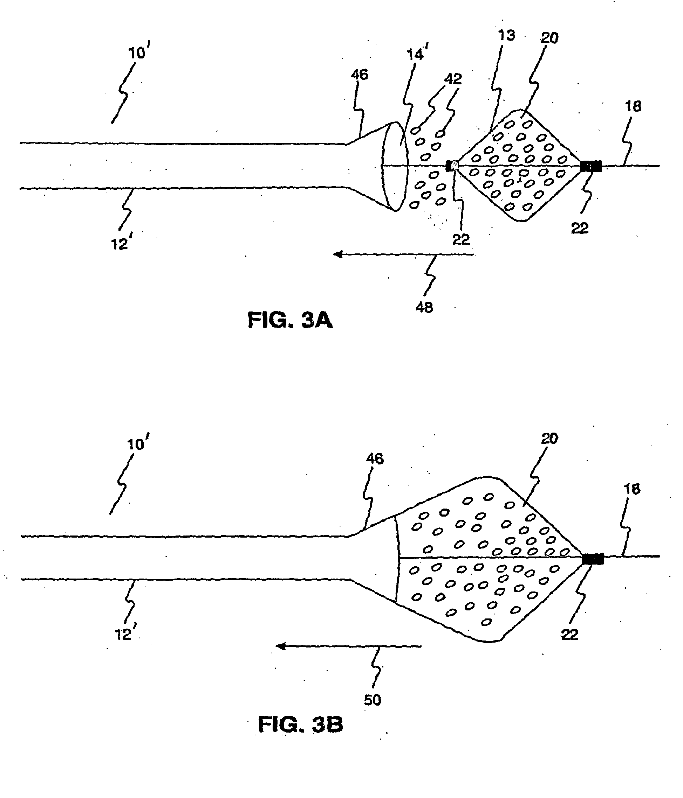

FIGS. 3A and 3B illustrate an additional embodiment of the present invention directed to a medical device 10' including an alternative sheath 12' for use with immobilization device 13. Sheath 12' is larger in comparison to previously described sheath 12 and includes a tapered distal end 46 and an enlarged distal opening 14'. During a medical procedure, the sheath 12 of FIGS. 1A to 2D may be completely removable from the elongate member 18 after deployment of the expansible member 20. In an alternative embodiment, not shown, the sheath 12 could be designed to tear away from the rest of the device 10, by the placement of a longitudinal split along the length of the sheath.

During use of the embodiment of FIGS. 3A-3B, after the initial sheath 12 is removed, larger sheath 12' is tracked over the proximal end of the elongate member 18 and advanced to facilitate encapsulation of the deployed and hydrated expansible member 20. The larger lumen and distal opening 14' of sheath 12' serve to more easily surround and collapse the deployed expansible member 20 upon retraction within the sheath 12'. In addition, the larger lumen and distal opening 14' can act as a retrieval device by trapping stones 42 within the lumen and/or between the sheath 12' and the expansible member 20, when the expansible member 20 is retracted proximally in the direction of arrow 48.

## Claims

1. A medical device, comprising:
a sheath (12) including a lumen (16), a distal end, and a proximal end; and
an elongate member (18) including an expansible member (20) connected to a distal portion (19) of the elongate member (18), the elongate member (18) and expansible member (20) being movable relative to the sheath (12) to achieve a first state of the expansible member (20) when the expansible member (20) is within the lumen (16) of the sheath (12) and an expanded state when the expansible member (20) extends from the distal end of the sheath (12), **characterized in that**
the expansible member (20) comprises a material that expands to the expanded state due to the presence of fluid, and
the material is flexible to adjust to contours of an anatomical lumen when the expansible member (20) is expanded to the expanded state.

2. The medical device of claim 1, wherein the first state is a compressed state.

3. The medical device of claim 1 or 2, wherein the elongate member (18) is a flexible wire.

4. The medical device of any one of claims 1 to 3, further comprising a handle at a proximal end of the medical device and configured to control axial movement of the expansible member (20) relative to the sheath (12).

5. The medical device of any one of claims 1 to 4, wherein the expansible member (20) has a proximal end and a distal end, and markers (22) are positioned along the elongate member (18) proximate the distal and proximal ends of the expansible member (20).

6. The medical device of any one of claims 1 to 5, wherein the expansible member (20) has a tapered proximal end to facilitate movement of the expansible member (20) into the lumen (16) of the sheath (12).

7. The medical device of any one of claims 1 to 6, wherein the expansible member (20) has a tapered distal end to facilitate movement of the expansible member (20) out of the lumen (16) of the sheath (12).

8. The medical device of any one of claims 1 to 7, wherein the expansible member (20) comprises a material that exhibits an expansion/compression size ratio of approximately 10:1.

9. The medical device of any one of claims 1 to 8, wherein the expansible member (20) comprises Poly-vinyl Alcohol (PVA).

10. The medical device of any one of claims 1 to 9, wherein the expansible member (20) comprises a sponge.

11. The medical device of any one of claims 1 to 10, wherein the expansible member (20) defines holes formed therein for passing irrigation therethrough in the expanded state.

12. The medical device of any one of claims 1 to 11, wherein the expansible member (20) comprises a material less susceptible to laser-energy induced damage than alloys of nickel/titanium, copper, cobalt, vanadium, chromium, and iron.

13. The medical device of any one of claims 1 to 12, wherein the sheath (12) defines a longitudinal split along the length such that the sheath (12) can be separated from the expansible member (20) and elongate member (18) by separation along the split.

14. The medical device of any one of claims 1 to 13, wherein the sheath (12) comprises a first sheath and the medical device further includes a second sheath for tracking over a proximal end of the elongate member (18) after removal of the first sheath, the second sheath having a lumen larger than the lumen of the first sheath and a distal end for receiving the expansible member (20).

15. The medical device of claim 14, wherein the second sheath includes a tapered distal end (46) extending to an enlarged distal opening (14').

## Patentansprüche

1. Medizinische Vorrichtung umfassend:
eine Hülle (12) mit einem Lumen (16), einem distalen Ende und einem proximalen Ende, und
ein langgestrecktes Element (18) mit einem ausdehnbaren Element (20), das mit einem distalen Abschnitt (19) des langgestreckten Elements (18) verbunden ist, wobei das langgestreckte Element (18) und das ausdehnbare Element (20) relativ zu der Hülle (12) beweglich sind, um einen ersten Zustand des ausdehnbaren Elements (20) zu erreichen, wenn das ausdehnbare Element (20) innerhalb des Lumens (16) der Hülle (12) ist, und einen expandierten Zustand, wenn das ausdehnbare Element (20) sich von dem distalen Ende der Hülle (12) erstreckt, **dadurch gekennzeichnet, daß**
das ausdehnbare Element (20) ein Material umfaßt, das sich in den expandierten Zustand aufgrund der Anwesenheit von Fluid ausdehnt, und
wobei das Material flexibel ist, um sich an Konturen eines anatomischen Lumens anzupassen, wenn das ausdehnbare Element (20) in den expandierten Zustand expandiert ist.

2. Medizinische Vorrichtung nach Anspruch 1, wobei der erste Zustand ein komprimierter Zustand ist.

3. Medizinische Vorrichtung nach Anspruch 1 oder 2, wobei das langgestreckte Element (18) ein flexibler Draht ist.

4. Medizinische Vorrichtung nach einem der Ansprüche 1 bis 3, ferner umfassend einen Griff an einem proximalen Ende der medizinischen Vorrichtung, der dazu ausgebildet ist, die axiale Bewegung des ausdehnbaren Elements (20) relativ zur Hülle (12) zu kontrollieren.

5. Medizinische Vorrichtung nach einem der Ansprüche 1 bis 4, wobei das ausdehnbare Element (20) ein proximales Ende und ein distales Ende besitzt und Markierungen (22) entlang des langgestreckten Elements (18) nahe des distalen und des proximalen Endes des ausdehnbaren Elements (20) positioniert sind.

6. Medizinische Vorrichtung nach einem der Ansprüche 1 bis 5, wobei das ausdehnbare Element (20) ein konisches proximales Ende besitzt, um die Bewegung des ausdehnbaren Elements (20) in das Lumen (16) der Hülle (12) zu erleichtern.

7. Medizinische Vorrichtung nach einem der Ansprüche 1 bis 6, wobei das ausdehnbare Element (20) ein konisches distales Ende besitzt, um die Bewegung des ausdehnbaren Elements (20) aus dem Lumen (16) der Hülle (12) zu erleichtern.

8. Medizinische Vorrichtung nach einem der Ansprüche 1 bis 7, wobei das ausdehnbare Element (20) ein Material mit einem Expansions/Kompressionsverhältnis von etwa 10:1 umfaßt.

9. Medizinische Vorrichtung nach einem der Ansprüche 1 bis 8, wobei das ausdehnbare Element (20) Poly-Vinyl Alkohol (PVA) umfaßt.

10. Medizinische Vorrichtung nach einem der Ansprüche 1 bis 9, wobei das ausdehnbare Element (20) einen Schwamm umfaßt.

11. Medizinische Vorrichtung nach einem der Ansprüche 1 bis 10, wobei das ausdehnbare Element (20) darin ausgebildete Hohlräume definiert, um im expandierten Zustand eine Spülung dort hindurch zu leiten.

12. Medizinische Vorrichtung nach einem der Ansprüche 1 bis 11, wobei das ausdehnbare Element (20) ein Material enthält, das weniger empfindlich gegen laserenergieinduzierte Beschädigung ist, als Legierungen aus Nickel/Titan, Kupfer, Kobalt, Vanadium, Chrom und Eisen.

13. Medizinische Vorrichtung nach einem der Ansprüche 1 bis 12, wobei die Hülle (12) eine längliche Teilung entlang der Hülle definiert, so daß die Hülle (12) von dem ausdehnbaren Element (20) und den verlängerten Element (18) durch Trennung entlang der Spalten separiert werden kann.

14. Medizinische Vorrichtung nach einem der Ansprüche 1 bis 13, wobei die Hülle (12) eine erste Hülle umfaßt und die medizinische Vorrichtung ferner eine zweite Hülle umfaßt, um über ein proximales Ende des verlängerten Elements (18) nach Entfernung der ersten Hülle zu folgen, wobei die zweite Hülle ein größeres Lumen als das Lumen der ersten Hülle und ein distales Ende zum Aufnehmen des ausdehnbaren Elements (20) besitzt.

15. Medizinische Vorrichtung nach einem der Ansprüche 1 bis 14, wobei die zweite Hülle ein konisches distales Ende (46) umfaßt, das sich zu einer erweiterten distalen Öffnung (14') ausdehnt.

## Revendications

1. Dispositif médical comprenant :
une gaine (12) incluant une lumière (16), une extrémité distale et une extrémité proximale, et
un élément allongé (18) incluant un élément expansible (20) relié à une partie distale (19) de l'élément allongé (18), l'élément allongé (18) et l'élément expansible (20) étant déplaçables par rapport à la gaine (12) pour obtenir un premier état de l'élément expansible (20) quand l'élément expansible (20) est à l'intérieur de la lumière (16) de la gaine (12) et un état expansé quand l'élément expansible (20) s'étend depuis l'extrémité distale de la gaine (12), **caractérisé en ce que**
l'élément expansible (20) comprend un matériau qui s'expanse jusqu'à l'état expansé du fait de la présence d'un fluide, et
le matériau est flexible pour s'ajuster aux contours d'une lumière anatomique quand l'élément expansible (20) est expansé jusqu'à l'état expansé.

2. Dispositif médical selon la revendication 1 où le premier état est un état compressé.

3. Dispositif médical selon la revendication 1 ou 2 où l'élément allongé (18) est un fil flexible.

4. Dispositif médical selon l'une quelconque des revendications 1 à 3 comprenant en outre une poignée à une extrémité proximale du dispositif médical et configurée pour commander le mouvement axial de l'élément expansible (20) par rapport à la gaine (12).

5. Dispositif médical selon l'une quelconque des revendications 1 à 4 où l'élément expansible (20) a une extrémité proximale et une extrémité distale, et des marqueurs (22) sont positionnés le long de l'élément allongé (18) à proximité des extrémités distale et proximale de l'élément expansible (20).

6. Dispositif médical selon l'une quelconque des revendications 1 à 5 où l'élément expansible (20) a une extrémité proximale effilée pour faciliter le mouvement de l'élément expansible (20) dans la lumière (16) de la gaine (12).

7. Dispositif médical selon l'une quelconque des revendications 1 à 6 où l'élément expansible (20) a une extrémité distale effilée pour faciliter le mouvement de l'élément expansible (20) hors de la lumière (16) de la gaine (12).

8. Dispositif médical selon l'une quelconque des revendications 1 à 7 où l'élément expansible (20) comprend un matériau qui présente un rapport des dimensions en expansion/compression d'approximativement 10:1.

9. Dispositif médical selon l'une quelconque des revendications 1 à 8 où l'élément expansible (20) comprend de l'alcool polyvinylique (PVA).

10. Dispositif médical selon l'une quelconque des revendications 1 à 9 où l'élément expansible (20) comprend une éponge.

11. Dispositif médical selon l'une quelconque des revendications 1 à 10 où l'élément expansible (20) définit des trous formés dans celui-ci pour faire passer une irrigation au travers de celui-ci dans l'état expansé.

12. Dispositif médical selon l'une quelconque des revendications 1 à 11 où l'élément expansible (20) comprend un matériau moins susceptible de détérioration induite par l'énergie laser que les alliages de nickel/titane, cuivre, cobalt, vanadium, chrome et fer.

13. Dispositif médical selon l'une quelconque des revendications 1 à 12 où la gaine (12) définit une fente longitudinale suivant la longueur de telle sorte que la gaine (12) peut être séparée de l'élément expansible (20) et de l'élément allongé (18) par séparation le long de la fente.

14. Dispositif médical selon l'une quelconque des revendications 1 à 13 où la gaine (12) comprend une première gaine et le dispositif médical inclut en outre une seconde gaine pour suivre et recouvrir une extrémité proximale de l'élément allongé (18) après retrait de la première gaine, la seconde gaine ayant une lumière plus grande que la lumière de la première gaine et une extrémité distale pour recevoir l'élément expansible (20).

15. Dispositif médical selon la revendication 14 où la seconde gaine inclut une extrémité distale effilée (46) s'étendant jusqu'à une ouverture distale agrandie (14').
